# DEMANDE DE BREVET EUROPEEN

(11) **EP 1 010 420 A1**
(43) Date de publication de la demande: **21.06.2000**
(21) Numéro de dépôt: 99402853.8
(22) Date de dépôt: 17.11.1999
(51) Int. Cl.: A61K 7/48, A61K 7/021

(54) **Composition cosmétique stable comprenant un copolymère particulier, des particules solides non enrobées et un polymère dispersant huileux**

(30) Priorité: 18.12.1998 FR 9816048
(71) Demandeur: L'OREAL, 75008 Paris (FR)
(72) Inventeur: Terren, Nadia, 92340 Bourg la Reine (FR); Favre, Sophie, 94550 Chevilly-Larue (FR)
(74) Mandataire: Brédeville, Odile Marie

(57) **Abrégé**

L'invention concerne une composition, notamment à usage cosmétique, dermato-logique, hygiénique et/ou pharmaceutique, comprenant au moins un copolymère particulier constitué de monomère acide carboxylique mono-oléfiniquement insaturé en C₃-C₆ et de monomère ester à chaîne grasse d'acide acrylique, des particules solides non enrobées et un polymère dispersant huileux. Elle concerne également les applications cosmétiques d'une telle composition.

## Description

La présente invention a pour objet une composition notamment à usage cosmétique, dermatologique, hygiénique ou pharmaceutique pouvant en particulier se présenter sous forme d'un gel, et susceptible d'être utilisée pour le soin et/ou le maquillage de la peau, des semi-muqueuses (intérieurs des paupières), des muqueuses (lèvres) et/ou des matières kératiniques (cheveux, cils, ongles).

Les compositions cosmétiques, notamment de maquillage telles que les rouges à lèvres, les anticernes ou les fonds de teint, comprennent généralement des corps gras tels que des huiles et des cires, et une phase particulaire généralement composée de charges et de pigments. Elles peuvent ainsi se présenter, par exemple dans le cas des rouges à lèvres, sous forme de stick ou bâton ou sous forme de pâte souple. Elles sont alors souvent sous la forme d'une composition anhydre. Les compositions de maquillage peuvent également comprendre de l'eau ou une phase hydrophile, et se présenter alors notamment sous forme d'émulsion huile-dans-eau, eau-dans-huile, émulsion multiple, solution ou gel aqueux, notamment lorsqu'il s'agit d'un fond de teint, de crème teintée, de crème de soin ou d'un produit solaire.

On a constaté que lorsque ces diverses compositions cosmétiques sont appliquées sur la peau, les muqueuses ou les semi-muqueuses, elles présentent l'inconvénient de transférer. On entend par là que la composition est susceptible de se déposer, au moins en partie, sur certains supports avec lesquels elle est mise en contact, tels que, par exemple, un verre, un vêtement ou la peau. En se déposant, ladite composition laisse une trace sur ledit support. Il s'en suit donc une persistance médiocre de la composition sur la peau ou les muqueuses, d'où la nécessité de renouveler régulièrement son application.

Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. Tous ces phénomènes engendrent un effet inesthétique, que la consommatrice souhaite bien évidemment éviter.

On a donc cherché à résoudre ces différents phénomènes de transfert et de migration. Dans le document EP-A-832 645, il est ainsi proposé d'introduire dans ces compositions un copolymère particulier constitué de monomère acide carboxylique mono-oléfiniquement insaturé en C₃-C₆ et de monomère ester à chaîne grasse d'acide acrylique La composition ainsi modifiée a un transfert limité, elle ne migre pas au cours du temps.

Toutefois, d'autres problèmes sont apparus et on a en particulier constaté que dans de telles compositions, l'utilisation de certaines particules solides, comme par exemple des pigments non enrobés, menait à l'obtention de compositions friables, instables, non lisses qui étaient inacceptables du point de vue cosmétique.

Or, il est intéressant en terme de formulation de pouvoir disposer par exemple de pigments non enrobés : en effet, l'enrobage des pigments est une opération généralement difficile, longue et coûteuse. De plus, certaines couleurs ne peuvent pas être obtenues à partir de pigments enrobés, la gamme de ces derniers étant limitée. L'utilisation de pigments non enrobés permet donc un plus grand choix de couleurs et donc des possibilités de formulations élargies. Elle permet également une fabrication industrielle plus facile et moins coûteuse.

Ainsi, il serait intéressant de pouvoir disposer de compositions, notamment cosmétiques et en particulier de maquillage, comprenant un copolymère particulier constitué de monomère acide carboxylique mono-oléfiniquement insaturé en C₃-C₆ et de monomère ester à chaîne grasse d'acide acrylique et des particules solides non enrobées, ces compositions restant par ailleurs stables et peu sujettes aux phénomènes de transfert et de migration.

La Demanderesse a trouvé de manière surprenante qu'en introduisant un polymère dispersant huileux dans une composition comprenant au moins un copolymère particulier constitué de monomère acide carboxylique mono-oléfiniquement insaturé en C₃-C₆ et de monomère ester à chaîne grasse d'acide acrylique et des particules solides non enrobées, il était possible d'obtenir une composition qui non seulement ne transférait pas mais qui de plus était particulièrement stable.

Ainsi, un des objets de la présente invention est une composition, notamment à usage cosmétique, dermatologique, hygiénique ou pharmaceutique, comprenant au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des particules solides non enrobées, caractérisée en ce qu'elle comprend en outre un polymère dispersant huileux.

Il est connu d'utiliser des polymères dispersants afin de disperser des pigments dans les peintures (voir l'article "Developments in Hyperdispersants Technology for Paints", Dr. J. Toole, ICI Organics Division, Paint & Resin, February 1985, page 25). Toutefois, rien ne laissait supposer que la présence d'un polymère dispersant huileux pourrait rendre compatibles un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique tel qu'utilisé dans la présente invention et des particules solides non enrobées, en particulier des pigments non enrobés, dans des compositions notamment cosmétiques et que l'on pourrait ainsi obtenir des compositions stables, non friables, et très peu sujettes aux phénomènes de transfert et de migration.

Ainsi, grâce à la présente invention, il est possible de réaliser des compositions cosmétiques, en particulier des gels émulsionnés, qui comprennent une proportion importante de pigments non enrobés et qui ne transfèrent pas ou très peu.

Les compositions selon l'invention répondent parfaitement aux normes de stabilité soit :
- résistance à l'épreuve de centrifugation à 900 g pendant 1 heure,
- résistance au vieillissement pendant 2 mois à température ambiante (25 °C) et ainsi qu'à 45 °C.

La composition selon l'invention répond aux critères suivants :
- elle a et conserve au cours de ces tests un aspect macroscopique et microscopique homogène, lisse et stable (globules finement dispersés, absence de relargage) et
- sa viscosité est constante au cours du temps.

Ces compositions sont de plus très faciles à fabriquer et peu coûteuses tout en conservant de bonnes propriétés cosmétiques : en effet, elles peuvent ne comprendre que des particules solides non enrobées, en particulier des pigments non enrobés, qui sont peu coûteuses. Elles ont l'avantage de s'étaler facilement sur la peau, elles sont douces, ne collent pas. Elles procurent également une sensation de fraîcheur à l'application. Bien entendu, elles ne transfèrent pas et ne migrent pas non plus, elles présentent une très bonne tenue au cours du temps. Le maquillage obtenu est homogène et présente un aspect naturel.

La présente invention a encore pour objet un procédé de traitement non thérapeutique de la peau et/ou du cuir chevelu, notamment un procédé de maquillage, consistant à appliquer sur la peau ou les muqueuses et/ou sur le cuir chevelu une composition cosmétique telle que définie ci-dessus.

La présente invention a également pour objet l'utilisation cosmétique d'un polymère dispersant huileux dans une composition comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des particules solides non enrobées dans le but de stabiliser ladite composition.

L'invention a encore pour objet l'utilisation d'un polymère dispersant huileux pour la préparation d'une composition comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des particules solides non enrobées dans le but de stabiliser ladite composition.

La présente invention a également pour objet l'utilisation d'un polymère dispersant huileux dans une composition stable et sans transfert comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des particules solides non enrobées.

L'invention a aussi pour objet un procédé pour stabiliser une composition comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des particules solides non enrobées consistant à introduire dans ladite composition un polymère dispersant huileux.

D'autres caractéristiques, aspects et avantages de la présente invention apparaîtront à la lecture de la description détaillée qui va suivre.

Les compositions selon l'invention comprennent au moins un copolymère constitué d'une fraction majoritaire de monomère acide carboxylique monooléfiniquement insaturé en C₃-C₆ ou de son anhydride, et d'une fraction minoritaire de monomère ester à chaîne grasse d'acide acrylique. Ce copolymère peut être éventuellement réticulé.

Le copolymère selon l'invention peut être préparé en polymérisant une quantité prépondérante de monomère carboxylique monooléfiniquement insaturé ou de son anhydride, avec une quantité plus faible de monomère ester acrylique à chaîne grasse. La quantité de monomère carboxylique ou de son anhydride est de préférence supérieure ou égale à 50 %, de préférence à 60 %, et de préférence encore comprise entre 80 et 98 % en poids et plus particulièrement entre 90 et 98 % en poids ; l'ester acrylique est de préférence présent dans des quantités inférieures ou égales à 50 %, de préférence à 40 %, et de préférence encore comprises entre 2 et 20 % en poids et plus particulièrement entre 2 et 10 % en poids ; les pourcentages sont calculés par rapport au poids total des deux monomères.

Les monomères carboxyliques préférentiels sont choisis parmi ceux répondant à la formule :

CH₂=CR-COOH

dans laquelle R désigne hydrogène, halogène, hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique.

Les monomères carboxyliques particulièrement préférés, sont choisis parmi l'acide acrylique, l'acide méthacrylique, l'anhydride maléique, et leurs mélanges.

Les monomères esters acryliques à chaîne grasse sont préférentiellement choisis parmi ceux répondant à la formule :

CH₂=CR¹-COOR²

dans laquelle R¹ est choisi dans le groupe formé par hydrogène, méthyle et éthyle et R² est un groupe alkyle en C₈-C₃₀, un groupe oxyalkylène en C₈-C₃₀, un groupe carbonyloxyalkylène en C₈-C₃₀.

Les monomères esters particulièrement préférés sont ceux pour lesquels R¹ est hydrogène ou méthyle, et/ou ceux pour lesquels R² est un groupe alkyle en C₁₀-C₂₂. On peut notamment citer les acrylates et méthacrylates de décyle, de lauryle, de stéaryle, de béhényle ou de mélissyle.

Certains des copolymères selon l'invention sont notamment décrits dans la demande EP-A-0268164 et sont obtenus selon les méthodes de préparation décrites dans ce même document.

On peut citer plus particulièrement les copolymères vendus sous le nom PEMULEN par la Société GOODRICH, et notamment le copolymère acrylate/C₁₀-C₃₀-alkylacrylate tel que le produit PEMULEN TR 2.

On peut, bien évidemment, utiliser un mélange de plusieurs copolymères tels que ci-dessus définis.

Ces copolymères peuvent être présents dans les compositions selon l'invention à une concentration de 0,01 à 3% en poids par rapport au poids total de la composition, de préférence 0,02 à 0,6% en poids, et plus préférentiellement de 0,05 à 0,2% en poids.

Les compositions selon l'invention comprennent des particules solides non enrobées. Par particules solides non enrobées, on entend, au sens de la présente invention, les pigments et/ou les nacres et/ou les charges habituellement utilisés dans les compositions cosmétiques et qui n'ont pas subi de traitement par des agents d'enrobage.

Par pigments, il faut comprendre des particules blanches ou colorées, minérales ou organiques, insolubles dans le milieu, destinées à colorer et/ou opacifier la composition. Les pigments peuvent être présents à raison de 0,1-30 % en poids, par rapport au poids total de la composition, et de préférence à raison de 2-20 %. Ils peuvent être blancs ou colorés, minéraux et/ou organiques.

Les pigments ont généralement une taille moyenne des particules élémentaires inférieure à 1 µm, de préférence inférieure à 500 nm. On peut aussi utiliser des nanopigments, dont la taille moyenne des particules élémentaires peut aller jusqu'à 100 nm et va de préférence de 5 à 100 nm.

On peut citer, parmi les pigments et les nanopigments minéraux, les dioxydes de titane, de zirconium ou de cérium, ainsi que les oxydes de zinc, les oxydes de fer (par exemple brun, jaune, rouge ou noir) ou de chrome, les nanotitanes, le bleu ferrique. Parmi les pigments organiques, on peut citer le noir de carbone, et les laques de baryum, strontium, calcium, aluminium.

Dans une forme préférée de réalisation de l'invention, les compositions comprennent des pigments non enrobés. De préférence encore, ces pigments non enrobés ont une taille moyenne des particules élémentaires inférieure à 500 nm. De préférence, les pigments non enrobés utilisés selon la présente invention sont des oxydes de fer de taille moyenne des particules élémentaires d'environ 300 nm. La teneur des pigments non enrobés peut aller de 2 à 30%, de préférence de 4 à 20% en poids, par rapport au poids total de la composition.

Par nacres, il faut comprendre des particules irisées qui réfléchissent la lumière.

Les nacres peuvent être présentes dans la composition à raison de 0-20% en poids, de préférence à un taux élevé de l'ordre de 2-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Par charges, il faut comprendre des particules incolores ou blanches, minérales ou de synthèse, lamellaires ou non lamellaires, destinées à donner de la douceur, de la matité et de l'uniformité au maquillage.

Les charges, qui peuvent être présentes dans la composition à raison de 0-20 % en poids, par rapport au poids total de la composition, de préférence 2-10%, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

De préférence, les particules solides non enrobées de la présente invention présentent une taille moyenne des particules élémentaires pouvant aller jusqu'à 1 micron. De préférence, les particules solides non enrobées sont présentes dans les compositions de l'invention à une teneur allant de 0,1 à 70% en poids, par rapport au poids total de la composition et de préférence encore de 0,1 à 40% en poids, par rapport au poids total de la composition.

Outre ces particules solides non enrobées, les compositions de l'invention peuvent également comprendre des particules solides enrobées. Les particules solides enrobées peuvent par exemple être des pigments et/ou des charges et/ou des nacres enrobés par des composés siliconés tels que des polydiméthylsiloxane et/ou par des polymères comme le polyéthylène ou encore par des acides aminés.

Dans une forme préférée de réalisation de l'invention, les compositions sont exemptes de pigments enrobés.

Les compositions selon l'invention comprennent un polymère dispersant huileux. Par polymère dispersant huileux, on entend, au sens de la présente invention, une molécule lipophile constituée d'une part d'une chaîne polymérique ayant une affinité pour un milieu lipophile et d'autre part une extrémité capable d'être absorbée sur la surface d'une particule solide.

De préférence, ces polymères dispersants huileux ont un poids moléculaire allant jusqu'à 15000, de préférence encore jusqu'à 12000, en poids moyen.

De tels dispersants huileux sont par exemple décrits dans l'article "Developments in Hyperdispersants Technology for Paints", Dr. J. Toole, ICI Organics Division, Paint & Resin, February 1985, page 25.

Les polymères dispersants huileux de la présente invention sont par exemple choisis parmi les polymères hydrocarbonés comportant au moins un hétéroatome O, N ou S comme les polymères d'acide hydroxystéarique, les polymères acrylamides et leurs dérivés, les polyacrylates modifiés lipophiles, les polydécènes, les copolymères d'acide acrylique et d'acrylate d'alkyle.

Des polymères dispersants huileux convenant à la présente invention sont par exemple le polyacrylate modifié dans le phtalate de di-iso-octyle à 50 % vendu sous la dénomination "EFKA 701" par la société EFKA, le polymère amphotère à longue chaîne vendu sous la dénomination "Tryosol 98 c" par la société TROY CHEMICAL, le copolymère d'acide acrylique et d'acrylate d'alkyle vendu sous la dénomination "Tegomer AC 100" par la société Goldschmidt.

Dans une forme préférée de l'invention, le polymère dispersant huileux est un polyacrylamide formé par condensation d'un acide polymérique et d'une amine puis élimination de l'eau ainsi formée, tel que décrit dans US 5, 480, 632.

L'acide polymérique peut être un polyester dérivé :
- d'un acide hydrocarboné de formule : HO - X - CO₂H où X est une chaîne carbonée aliphatique saturée ou insaturée éventuellement interrompue par un atome O, N ou S et comprenant de préférence de 8 à 250 atomes de carbone, de préférence encore de 12 à 50 atomes de carbone et dans laquelle il y a au moins 4 atomes, de préférence 4 atomes de carbone, entre le radical hydroxy et le radical carboxylique,
- ou d'un mélange d'un acide carboxylique hydroxylé tel que ci-dessus et d'un acide carboxylique exempt de groupe hydroxy.

Les hydroxyacides de formule HO - X - CO₂H préférés sont les acides alcanoïques en C₁₂-C₂₀ hydroxylés.

Les acides gras hydroxylés tels que l'acide hydroxystéarique décrit dans US 4,349,389 sont préférés.

Les amines utilisées pour former le polyamide sont des alkylamines ou des polyamines comme par exemple la méthylamine, la diéthylamine, la triéthylamine, la diméthyl-aminopropylamine, l'éthylènediamine, la triéthylène-tétramine, la guanidine et leurs dérivés.

Les polymères dispersants huileux sont de préférence les polymères d'acide hydroxystéarique vendus sous les dénominations Solsperse par la Société ZENECA, en particulier le "Solsperse 21000".

Le polymère dispersant huileux est généralement présent dans les compositions selon l'invention à une teneur allant de 0,001 à 5 %, en poids, de préférence de 0,01 à 3%, par rapport au poids total de la composition.

Dans une forme préférée de réalisation de l'invention, les compositions comprennent en outre un polymère poly(acide 2-acrylamido 2-méthylpropane sulfonique) réticulé et neutralisé à au moins 90%.

Ce polymère poly(acide 2-acrylamido 2-méthylpropane sulfonique) réticulé et pratiquement ou totalement neutralisé est généralement hydrosoluble ou gonflable dans l'eau. Il peut être caractérisé par le fait qu'il comprend, distribués de façon aléatoire :
a) de 90 à 99,9% en poids de motifs de formule générale (1) suivante : 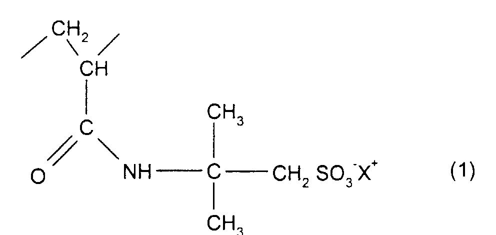 dans laquelle X⁺ désigne un cation ou un mélange de cations, au plus 10% molaire des cations X⁺ pouvant être des protons H⁺ ; et
b) de 0,01 à 10% en poids de motifs réticulants provenant d'au moins un monomère ayant au moins deux doubles liaisons oléfiniques ; les proportions en poids étant définies par rapport au poids total du polymère.

Ce polymère comprend de préférence de 98 à 99,5 % en poids de motifs de formule (1) et de 0,2 à 2 % en poids de motifs réticulants.

Le cation X⁺ représente un cation ou un mélange de cations choisis en particulier parmi un proton, un cation de métal alcalin, un cation équivalent de celui d'un métal alcalino-terreux ou l'ion ammonium. Le cation X⁺ préférentiel est le cation NH₄⁺. Plus particulièrement, 90 à 100% mole des cations sont des cations NH₄⁺ et 0 à 10% mole sont des protons (H⁺).

Les monomères de réticulation ayant au moins deux doubles liaisons oléfiniques sont choisis par exemple parmi le dipropylèneglycol-diallyléther, les polyglycoldiallyléthers, le triéthylèneglycol-divinyléther, l'hydroquinone-diallyl-éther, le tétrallyl-oxéthanoyle ou d'autres allyl ou vinyléthers alcools polyfonctionnels, le diacrylate de tétraéthylèneglycol, la triallylamine, le triméthylolpropane-diallyléther, le méthylen-bis-acrylamide ou le divinylbenzène.

Les monomères de réticulation ayant au moins deux doubles liaisons oléfiniques sont plus particulièrement choisis parmi ceux répondant à la formule générale (2) suivante : 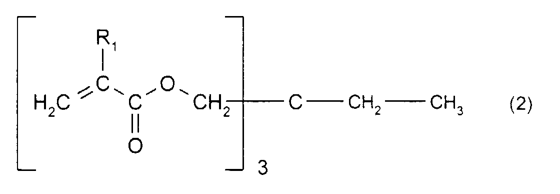 dans laquelle R₁ désigne un atome d'hydrogène ou un radical alkyle en C₁-C₄ et plus particulièrement le radical méthyle (triméthylol propane triacrylate).

Les polymères particulièrement préférés sont ceux présentant une viscosité mesurée au viscosimètre BROOKFIELD, mobile 4, à une vitesse de rotation de 100 tours/minutes, à 25°C, et en solution aqueuse à 2 % en poids, supérieure ou égale à 1000 cps (1000 mPa.s) et plus préférentiellement allant de 5000 cps à 40000 cps (5000 à 40000 mPa.s) et plus particulièrement de 6500 cps à 35000 cps.

Les poly(acide 2-acrylamido 2-méthylpropane sulfonique) réticulés, pratiquement ou totalement neutralisés peuvent être présents dans les compositions selon l'invention à une concentration de 0,01 à 20% en poids par rapport au poids total de la composition, de préférence 0,1 à 5% en poids, et plus préférentiellement 0,4 à 2% en poids.

Les compositions selon l'invention peuvent aussi comprendre des dérivés polycarboxyvinyliques du type Carbomer (vendus par GOODRICH sous les dénominations Carbopol 910, 934, 940, 941, 934 P, 980 ou par 3V-SIGMA sous la dénomination Synthalen K ou Synthalen L). Ces dérivés polycarboxyvinyliques peuvent être présents dans les compositions selon l'invention à une teneur allant de 0,01 à 5%, de préférence de 0,1 à 3% en poids, par rapport au poids total de la composition.

Les compositions selon l'invention peuvent également comprendre des épaississants tels que les polymères et les copolymères réticulés d'acrylamide, tels que ceux vendus sous les dénominations de "PAS 5161" ou "Bozepol C" par la Société Hoechst, de "Sepigel 305" par la Société Seppic, ou de "Salcare SC95" par la Société Allied Colloïd.

Les compositions de l'invention contiennent en outre un milieu cosmétiquement, hygiéniquement, pharmaceutiquement ou dermatologiquement acceptable, c'est-à-dire un milieu compatible avec toutes les matières kératiniques telles que la peau, les ongles, les cheveux, les cils et sourcils, les muqueuses et les semi-muqueuses, et toute autre zone cutanée du corps et du visage.

Les compositions de l'invention peuvent par exemple se présenter sous la forme d'une émulsion huile-dans-eau ou d'une émulsion multiple. Dans une forme préférée de réalisation, la composition selon l'invention se présente sous la forme d'un gel émulsionné.

La phase aqueuse peut alors comprendre de l'eau, une eau florale telle que l'eau de bleuet et/ou une eau minérale telle que l'eau de VITTEL, l'eau de LUCAS ou l'eau de LA ROCHE POSAY.

Ladite phase aqueuse peut être présente à une teneur de 15 à 99,5 % en poids par rapport au poids total de la composition, de préférence 40 à 80% en poids lorsque la composition se présente sous forme d'émulsion huile-dans-eau, ou de préférence 85 à 95% en poids lorsque la composition se présente sous la forme d'un gel.

En outre, la phase aqueuse peut comprendre de 0 % à 14 % en poids, par rapport au poids total de la phase aqueuse, d'un monoalcool inférieur en C₂-C₆ et/ou d'un polyol tel que le glycérol, le butylèneglycol, l'isoprène glycol, le propylèneglycol, le polyéthylèneglycol.

La phase grasse des compositions selon l'invention peut comprendre, outre le polymère dispersant huileux cité ci-dessus, des corps gras liquides à 25°C, tels que des huiles d'origine animale, végétale, minérale ou synthétique.

Lorsque la composition selon l'invention se présente sous la forme d'une émulsion, ladite phase grasse peut comprendre toute huile cosmétiquement acceptable, dans la mesure où ladite huile permet, en mélange avec la phase aqueuse et les éventuels additifs, l'obtention d'une émulsion stable, c'est-à-dire d'une émulsion qui ne casse pas, qui reste sous forme d'une phase unique pendant au moins 24 heures après stockage à 25°C, sans phénomène de crémage ou de relarguage d'huile.

Les huiles susceptibles d'être employées peuvent éventuellement être volatiles.

On entend par huile volatile, tout composé susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.

On peut ainsi citer les huiles siliconées volatiles, telles que :
- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE , qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane, de l'hexyl heptaméthyltrisiloxane ou de l'octyl heptaméthyltrisiloxane.
On peut également citer les huiles volatiles hydrocarbonées, telles que les isoparaffines et notamment l'isododécane.

Parmi les huiles non volatiles, on peut citer :
- les polyalkyl(C₁-C₂₀) siloxanes et notamment ceux à groupements terminaux triméthylsilyle, de préférence ceux dont la viscosité est inférieure à 0,06 m²/s parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées, notamment celles de formule : 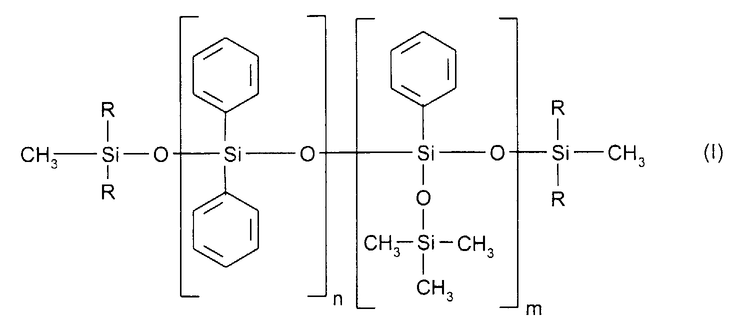 dans laquelle R est un radical alkyle en C1-C30, un radical aryle ou un radical aralkyle, n est un nombre entier compris entre 0 et 100, et m est un nombre entier compris entre 0 et 100, sous réserve que la somme est comprise entre 1 et 100.
- les huiles d'origine animale, végétale ou minérale, telles que l'huile de paraffine, de vaseline, le perhydrosqualène, l'huile d'abricot, l'huile de germes de blé, d'amande douce, de calophyllum, de sésame, de macadamia, de pépins de raisin, de colza, de coprah, d'arachide, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides, les huiles fluorées et perfluorées.

Lorsque la composition se présente sous forme d'une émulsion huile-dans-eau, la phase grasse de l'émulsion peut être présente à une teneur de 2 % à 40 % en poids par rapport au poids total de l'émulsion, de préférence de 3 % à 30 % en poids et en particulier de 3 % à 20 % en poids.

La composition selon l'invention peut comprendre en outre d'autres corps gras, qui peuvent être choisis par l'homme du métier sur base de ses connaissances générales, de manière à conférer à la composition finale les propriétés souhaitées, par exemple en consistance, en texture et/ou en transfert. Ces corps gras additionnels peuvent être des cires, des gommes et/ou des corps gras pâteux d'origine animale, végétale, minérale ou synthétique, ainsi que leurs mélanges.

On peut notamment citer :
- les gommes de silicones,
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

La composition selon l'invention peut en outre comprendre un ou plusieurs solvants organiques cosmétiquement acceptables (tolérance, toxicologie et toucher acceptables). Ces solvants organiques peuvent représenter de 0 % à 98 % du poids total de la composition. Ils peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des monoalcools inférieurs linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer des polyols tels des dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

La composition selon l'invention peut éventuellement comprendre en outre un tensioactif, bien que cela ne soit pas nécessaire pour l'obtention d'une émulsion stable.

Elle peut également comprendre 0 à 5 % en poids, par rapport au poids total de l'émulsion, d'au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition peut comprendre des colorants hydrosolubles choisis parmi les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition peut comprendre en outre tout composé complémentaire usuellement utilisé dans le domaine cosmétique, tel que des antioxydants, des parfums, des huiles essentielles, des conservateurs, des actifs cosmétiques, des hydratants, des vitamines, des acides gras essentiels, des sphingolipides, des composés auto-bronzants, des filtres solaires, des polymères liposolubles notamment hydrocarbonés, tels que le polybutène, les polyalkylènes, les polyacrylates et les polymères siliconés compatibles avec les corps gras.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Ces composés complémentaires peuvent être présents dans la composition à raison de 0-10% en poids.

Les compositions selon l'invention peuvent se présenter sous toutes les formes appropriées pour une application topique, notamment sous forme de lotion, de crème éventuellement gélifiée, de lait, de gel, d'émulsions obtenues par dispersion d'une phase grasse dans une phase aqueuse (H/E) de consistance liquide ou semi-liquide, voire pâteuse ou solide.

Les compositions selon l'invention trouvent une application notamment dans le domaine du maquillage de la peau, des semi-muqueuses, des muqueuses et/ou des phanères, et se présentent alors par exemple sous la forme d'un fond de teint, d'un anticerne, d'un produit de maquillage pour le corps, d'un fard à joues ou à paupières, d'un rouge à lèvres, d'un mascara ou d'un eye-liner.

Elles peuvent également être utilisées comme base de soin pour les lèvres, ou comme produit de soin de la peau, des muqueuses, des semi-muqueuses et/ou des phanères, produit hygiénique ou pharmaceutique ou encore produit solaire ou autobronzant.

Les compositions selon l'invention peuvent être préparées selon des méthodes classiques de préparation des compositions bien connues de l'homme du métier, en particulier les méthodes de préparation des émulsions et des gels émulsionnés.

Elles trouvent également une application dans le domaine capillaire, notamment en tant que gels ou crèmes de soin des fibres kératiniques telles que cheveux, cils et sourcils, ou encore en tant que gel aqueux notamment de coiffage.

L'invention est illustrée plus en détails dans l'exemple suivant.

### EXEMPLE :

La Demanderesse a réalisé les compositions suivantes (les quantités sont données en pourcentage de poids par rapport au poids total de la composition) :

### Composition 1 :

### Phase A :

- pigments non enrobés 10,4%
- acide poly-hydroxystéarique vendu sous la dénomination commerciale "Solsperse 21000" par la société Zeneca 0,36 %
- phényltriméthicone 15,24%

### Phase B :

- copolymère acrylate/C₁₀-C₃₀-alkylacrylate vendu sous la dénomination commerciale "Pemulen TR 2" par la société Goodrich 0,1 %
- carbomer vendu sous la dénomination commerciale "Carbopol 980" par la société Goodrich 0,6%
- triéthanolamine 0,8%

### Phase C :

- conservateurs 0,5%
- eau qsp 100%

Cette composition a été réalisée de la manière suivante : on prépare dans un premier temps la phase A. Puis on disperse la phase B dans la phase C (eau).

Puis on disperse la phase A dans (B + C). Cette composition est donc très facile à fabriquer. Elle est exempte de pigments enrobés et est donc très peu coûteuse.

De plus, la composition ainsi obtenue est stable au cours du temps, en particulier elle ne montre aucun déphasage après 2 mois à 45°C.

Enfin, cette composition présente l'avantage de transférer très peu.

### Composition 2 :

### Phase A :

- pigments non enrobés (oxydes de fer brun, jaune) 14 %
- acide poly-hydroxystéarique vendu sous la dénomination commerciale "Solsperse 21000" par la société Zeneca 0,49 %
- phényltriméthicone 20,51%

### Phase B :

- copolymère acrylate/C₁₀-C₃₀-alkylacrylate vendu sous la dénomination commerciale "Pemulen TR 2" par la société Goodrich 0,1 %
- poly(acide 2-acrylamido 2-méthylpropane sulfonique) réticulé et neutralisé à 90% 0,5 %

### Phase C :

- eau 64,4 %

Cette composition a été réalisée de la manière suivante : on prépare dans un premier temps la phase A. Puis on disperse la phase B dans la phase C (eau). Puis on disperse la phase A dans (B + C). Cette composition est donc très facile à fabriquer. Elle est exempte de pigments enrobés et est donc très peu coûteuse.

De plus, la composition ainsi obtenue est stable au cours du temps, en particulier elle ne montre aucun déphasage après 2 mois à 45°C.

Enfin, cette composition présente l'avantage de transférer très peu.

La Demanderesse a également réalisé une composition 3 identique à la composition 2 ci-dessus mais ne comprenant pas l'acide poly-hydroxystéarique. La composition 3 est friable, cassante. Elle n'est pas lisse et n'est pas acceptable cosmétiquement.

### Composition 4 :

### Phase A :

- pigments non enrobés 10,4%
- copolymère d'acide acrylique et d'acrylate d'alkyle vendu sous la dénomination commerciale "Tegomer AC 100" par la société Goldschmidt 0,36 %
- phényltriméthicone 15,24%

### Phase B :

- copolymère acrylate/C₁₀-C₃₀-alkylacrylate vendu sous la dénomination commerciale "Pemulen TR 2" par la société Goodrich 0,1 %
- carbomer vendu sous la dénomination commerciale "Carbopol 980" par la société Goodrich 0,6%
- triéthanolamine 0,8%

### Phase C :

- conservateurs 0,5%
- eau qsp 100%

Cette composition a été réalisée de la manière suivante : on prépare dans un premier temps la phase A. Puis on disperse la phase B dans la phase C (eau). Puis on disperse la phase A dans (B + C). Cette composition est donc très facile à fabriquer. Elle est exempte de pigments enrobés et est donc très peu coûteuse.

De plus, la composition ainsi obtenue est stable au cours du temps, en particulier elle ne montre aucun déphasage après 2 mois à 45°C.

Enfin, cette composition présente l'avantage de transférer très peu.

## Revendications

1. Composition comprenant au moins un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, et des particules solides non enrobées, caractérisée en ce qu'elle comprend en outre un polymère dispersant huileux.

2. Composition selon la revendication 1, caractérisée par le fait que :
- le monomère acide carboxylique est choisi parmi les composés de formule CH₂=CR-COOH dans laquelle R désigne hydrogène, halogène, hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique,
- et le monomère ester à chaîne grasse d'acide acrylique est choisi parmi les composés de formule CH₂=CR¹-COOR² dans laquelle R¹ est choisi dans le groupe formé par hydrogène, méthyle et éthyle et R² est un groupe alkyle en C₈-C₃₀, un groupe oxyalkylène en C₈-C₃₀, un groupe carbonyloxyalkylène en C₈-C₃₀.

3. Composition selon la revendication 2, caractérisée par le fait que :
- le monomère acide carboxylique est choisi parmi l'acide acrylique, l'acide méthacrylique, l'anhydride maléique, et leurs mélanges,
- et le monomère ester à chaîne grasse d'acide acrylique est choisi parmi les acrylates et méthacrylates de décyle, de lauryle, de stéaryle, de béhényle ou de mélissyle.

4. Composition selon la revendication 2 ou 3, caractérisée par le fait que le copolymère est un copolymère acrylate/C₁₀-C₃₀-alkylacrylate.

5. Composition selon l'une quelconque des revendications 1 à 4, caractérisée par le fait que le copolymère est présent dans la composition à une concentration de 0,01 à 3% en poids, de préférence 0,02 à 0,6% en poids, et plus préférentiellement de 0,05 à 0,2% en poids, par rapport au poids total de la composition.

6. Composition selon l'une quelconque des revendications précédentes, caractérisée par le fait que les particules solides non enrobées sont des pigments non enrobés.

7. Composition selon la revendication précédente, caractérisée par le fait que les pigments non enrobés ont une taille moyenne des particules élémentaires inférieure à 500 nm.

8. Composition selon la revendication 6 ou 7, caractérisée par le fait que les pigments non enrobés sont choisis parmi les dioxydes de titane, de zirconium ou de cérium, les oxydes de zinc, de fer ou de chrome, les nanotitanes, le bleu ferrique, le noir de carbone, les laques de baryum, strontium, calcium, ou aluminium.

9. Composition selon la revendication précédente, caractérisée par le fait que les pigments non enrobés sont des oxydes de fer.

10. Composition selon l'une quelconque des revendications 6 à 9, caractérisée par le fait que les pigments non enrobés sont présents dans la composition à une teneur allant de 2 à 30% en poids, de préférence de 4 à 20% en poids, par rapport au poids total de la composition.

11. Composition selon l'une quelconque des revendications précédentes, caractérisée par le fait que les particules solides non enrobées sont présentes à une teneur allant de 0,1 à 70%, de préférence de 0,1 à 40% en poids, par rapport au poids total de la composition.

12. Composition selon l'une quelconque des revendications précédentes, caractérisée par le fait qu'elle est exempte de pigments enrobés.

13. Composition selon l'une quelconque des revendications précédentes, caractérisée par le fait que le polymère dispersant huileux est choisi parmi les polymères d'acide hydroxystéarique, les polymères acrylamides et leurs dérivés, les polyacrylates modifiés lipophiles, les polydécènes.

14. Composition selon l'une quelconque des revendications précédentes, caractérisée par le fait que le polymère dispersant huileux est un polymère d'acide hydroxystéarique.

15. Composition selon l'une quelconque des revendications précédentes, caractérisée par le fait que le polymère dispersant est présent dans la composition à une teneur allant de 0,001 à 5%, en poids, par rapport au poids total de la composition, de préférence de 0,01 à 3% en poids, par rapport au poids total de la composition.

16. Composition selon l'une quelconque des revendications précédentes, caractérisée par le fait qu'elle comprend en outre un polymère poly(acide 2-acrylamido 2-méthylpropane sulfonique) réticulé et neutralisé à au moins 90%.

17. Composition selon la revendication 16, caractérisée par le fait que ledit polymère est présent dans la composition à une teneur allant de 0,01 à 20% en poids, de préférence 0,1 à 5% en poids, et plus préférentiellement 0,4 à 2% en poids, par rapport au poids total de la composition.

18. Composition selon l'une quelconque des revendications précédentes, caractérisée par le fait qu'elle est à usage cosmétique, dermatologique, hygiénique ou pharmaceutique.

19. Composition selon l'une quelconque des revendications précédentes, caractérisée par le fait qu'elle se présente sous la forme d'un gel émulsionné.

20. Composition selon l'une quelconque des revendications précédentes, caractérisée par le fait qu'elle se présente sous la forme d'un fond de teint, d'un anticerne, d'un produit de maquillage pour le corps, d'un fard à joues ou à paupières, d'un rouge à lèvres, d'un mascara ou d'un eye-liner.

21. Composition selon l'une quelconque des revendications précédentes, caractérisée par le fait qu'elle est stable.

22. Procédé de traitement non thérapeutique de la peau et/ou du cuir chevelu, notamment un procédé de maquillage, consistant à appliquer sur la peau ou les muqueuses et/ou sur le cuir chevelu une composition cosmétique telle que définie à l'une quelconque des revendications 1 à 21.

23. Utilisation cosmétique d'un polymère dispersant huileux dans une composition comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, et des particules solides non enrobées, en particulier des pigments non enrobés, dans le but de stabiliser ladite composition.

24. Utilisation d'un polymère dispersant huileux pour la préparation d'une composition comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, et des particules solides non enrobées, en particulier des pigments non enrobés, dans le but de stabiliser ladite composition.

25. Procédé pour stabiliser une composition comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, et des particules solides non enrobées, en particulier des pigments non enrobés, consistant à introduire dans ladite composition un polymère dispersant huileux.

26. Utilisation d'un polymère dispersant huileux dans une composition stable et sans transfert comprenant un copolymère, éventuellement réticulé, constitué d'une fraction majoritaire de monomère acide carboxylique monooléfiniquement insaturé en C₃-C₆ ou de son anhydride, et d'une fraction minoritaire de monomère ester à chaîne grasse d'acide acrylique et des particules solides non enrobées, en particulier des pigments non enrobés.
